# EUROPEAN PATENT APPLICATION

(11) **EP 0 710 665 A1**
(43) Date of publication of application: **08.05.1996**
(21) Application number: 95307618.9
(22) Date of filing: 26.10.1995
(51) Int. Cl.: C07F 9/40, A61K 31/66

(54) **Phosphonosulfonate squalene synthetase inhibitor salts and method**

(30) Priority: 03.11.1994 US 19940333661
(71) Applicant: BRISTOL-MYERS SQUIBB COMPANY, Princeton, NJ 08543-4000 (US)
(72) Inventor: Pendri, Yadagiri, Matawan, NJ (US); Chen, Chung-Pin, Kendall Park, NJ (US); Kucera, David J., Warren, NJ (US); Martinez, Eduardo J., New Brunswick, NJ (US); Pansegrau, Paul D., N.W., Beulah, ND (US); Thottathil, John K., Robbinsville, NJ (US); Timmins, Peter, Wirral, Merseyside, L61 4XD (GB)
(74) Representative: Thomas, Roger Tamlyn, et al

(57) **Abstract**

New salt forms of the phosphonosulfonate squalene synthetase inhibitor 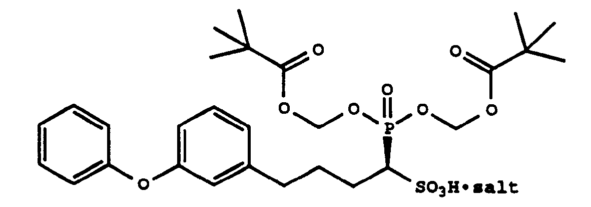 are provided which include the calcium, the t-butylamine salt, t-octylamine salt and the dehydroabietylamine salt. These salts inhibit cholesterol biosynthesis and therefore are used in lowering serum cholesterol and in treating atherosclerosis.

## Description

The present invention relates to new salt forms of the phosphonosulfonate squalene synthetase inhibitor 3-phenoxy-α-phosphonobenzenebutanesulfonic acid, dipivaloyloxy methyl ester, which include amine salts such as t-butylamine salt, t-octylamine salt and the dehydroabietylamine salt, and to their use in inhibiting de novo cholesterol biosynthesis, in lowering serum cholesterol and in treating atherosclerosis.

European Patent Application No. 0595635A1 discloses α-phosphonosulfonate compounds which inhibit cholesterol biosynthesis, and thus are useful as hypocholesterolemic and antiatherosclerotic agents and have the following structure 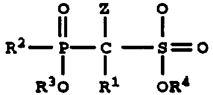 wherein R² is OR⁵ or R^{5a}, R³ and R⁵ are the same or different and are H, alkyl, arylalkyl, aryl, cycloalkyl, a metal ion or other pharmaceutically acceptable cation as defined below, or a prodrug ester;
R^{5a} is H, alkyl, arylalkyl or aryl;
R⁴ is H, alkyl, cycloalkyl, aryl, arylalkyl, metal ion or other pharmaceutically acceptable cation as defined below, or a prodrug ester;
Z is H, halogen, lower alkyl or lower alkenyl;
R¹ is a lipophilic group containing at least 7 carbons and is alkyl containing from 7 to 25 carbons in the chain; alkenyl containing from 7 to 25 carbons in the chain and from 1 to 6 double bonds; alkynyl containing from 7 to 25 carbons in the chain and from 1 to 6 triple bonds; mixed alkenyl-alkynyl containing 1 to 5 double bonds and 1 to 5 triple bonds; cycloalkyl; cycloheteroalkyl linked through a carbon on the ring or a heteroatom; aryl; heteroaryl; heteroarylalkyl; cycloalkylalkyl;
cycloheteroalkylalkyl; or a group of the structure 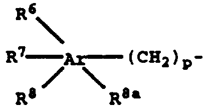
wherein Ar is aryl (such as phenyl or naphthyl), heteroaryl (5 or 6 membered) and may include one to three additional rings fused to Ar (such as aryl, cycloalkyl, heteroaryl or cycloheteroalkyl) and wherein (CH₂)ₚ contains from 1 to 15 carbons, preferably 2 to 12 carbons,
in the chain and may include 0, 1, 2 or 3 double bonds and/or 0, 1, 2 or 3 triple bonds in the normal chain, and may contain an ether or amino function in the chain, and/or may include 0, 1, 2 or 3 substituents as defined below for R⁶; and R⁶, R⁷, R⁸ and R^{8a} are the same or different and are H, alkyl containing 1 to 40 carbons, preferably from 3 to 25 carbons, alkoxy containing 1 to 40 carbons,
preferably from 3 to 25 carbons, alkenyl containing 2 to 40 carbons, preferably from 3 to 25 carbons, alkenyloxy containing 2 to 40 carbons, preferably from 3 to 25 carbons, alkynyl containing 2 to 40 carbons, preferably from 3 to 25 carbons, alkynyloxy containing 2 to 40 carbons, preferably from 3 to 25 carbons, cycloheteroalkyl, cycloheter-oalkylalkyl, heteroaryl, cycloalkyl, cycloalkylalkyl, Ar-alkyl, (such as arylalkyl), ArO (such as aryloxy), Ar-amino (such as arylamino), hydroxy, halogen, nitro, Ar (such as aryl), amino, substituted amino wherein the amino includes 1 or 2 substituents (which are alkyl, alkenyl, aryl or any of the Ar groups mentioned above), thiol, alkylthio, Ar-thio (such as arylthio), alkyl-sulfinyl, Ar-sulfinyl (such as arylsulfinyl), alkylsulfonyl, Ar-sulfonyl (such as arylsulfonyl), carboxy, cyano, alkoxycarbonyl, aminocarbonyl, alkylcarbonyloxy, Ar-carbonyloxy (such as arylcarbonyloxy), Arcarbonylamino (such as arylcarbonylamino) or alkylcarbonylamino, as well as any of the Ar groups as defined above, and preferably wherein the total number of carbons in the substituted Ar-(CH₂)ₚ- group exceeds 10 carbons; including pharmaceutically acceptable salts thereof such as alkali metal salts such as lithium, sodium or potassium, alkaline earth metal salts such as calcium or magnesium, as well as zinc or aluminum and other FDA approved cations such as ammonium, choline, diethanolamine, ethylenediamine, and salts of naturally occuring amino acids such as arginine, lysine, alanine and the like.

The (CH₂)ₚ group may contain 1, 2, 3 or more alkyl, alkoxy, alkenyl, alkynyl, hydroxy and/or halogen substituents as well as any of the substituents defined for R⁶.

The term "prodrug esters" as employed in the European Patent Application No. 0595635A1 includes prodrug esters which are known in the art for both phosphorus and carboxylic acids. Examples include the following groups: (I-alkanoyloxy)alkyl such as, 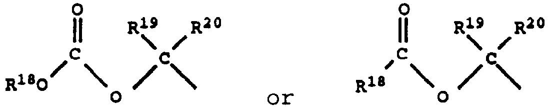 wherein R¹⁸, R¹⁹ and R²⁰ are H, alkyl, aryl or arylalkyl; however R¹⁸O cannot be HO. Examples of such prodrug esters include
CH₃CO₂CH₂-,

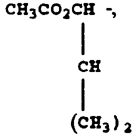

t-C₄H₉CO₂CH₂-, or

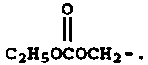

European Patent Application No. 0595635A1 also discloses the preparation of the squalene synthetase inhibitor 3-phenoxy-α-phosphonobenzene-butanesulfonic acid tripotassium salt, including enantiomers thereof, which has the structure

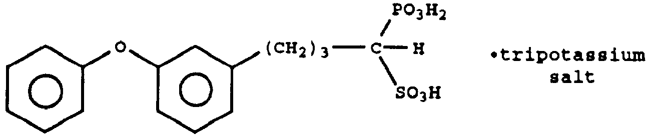

In accordance with the present invention, phosphonosulfonate salts are provided which have the structure I 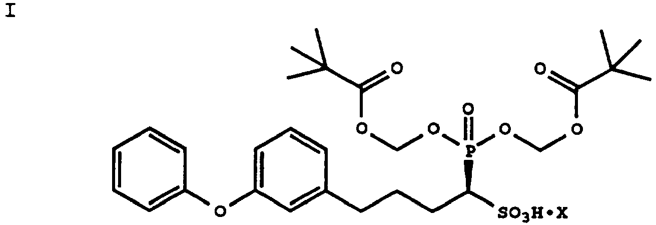 wherein X is a salt which is the t-butylamine salt, the t-octylamine salt or the dehydroabietylamine salt.

Preferred is the t-butylamine salt.

It has been found that the above salts are water-soluble, non-hygroscopic, exhibit a high degree of crystallinity, have good shelf life and provide good oral bioavailability of the squalene synthetase inhibitor.

The starting material employed is 3-phenoxy-α-phosphonobenzenebutanesulfonic acid 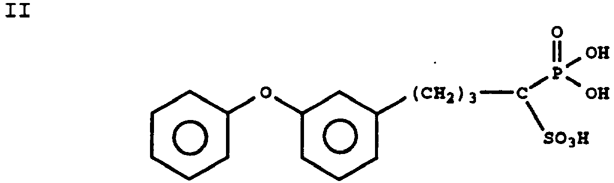 preferably the (S) enantiomer (prepared as described in European Patent Application No. 93308617.5).

In addition, in accordance with the present invention, a method is provided for isolating the free acid II in substantially pure form for use in preparing calcium salt III as described hereinafter. Thus, one aspect of the purification method of the invention includes the steps of forming crystalline salts of crude free acid II with 1-adamantanamine or cinchonidine to form the corresponding bis-adamantanamine salt of the free acid, or the bis-cinchonidine salt of the free acid, and subjecting the bis-adamantanamine salt or bis-cinchonidine salt to cation-exchange to afford purified free acid.

In carrying out the above purification procedure, the crude free acid will preferably be dissolved in an alcohol solvent such as methanol, ethanol or isopropanol, preferably methanol while the 1-adamantanamine and cinchonidine will be dissolved in a suitable organic solvent such as ethyl acetate, diethyl ether, tetrahydrofuran, dichloromethane, acetonitrile or toluene.

The solution of cinchonidine is reacted with the solution of crude free acid at a temperature within the range of from about 15 to about 80°C, preferably from about 50 to about 70°C and the resulting reaction mixture is cooled to precipitate the bis-cinchonidine salt.

In a further aspect of the present invention, purified free acid compound II is dissolved in an inert organic solvent such as anhydrous dimethylformamide (DMF), or dimethylsulfoxide (DMSO), acetonitrile, ethyl acetate, dichloromethane, toluene or other inert organic solvent, and then treated with iodomethyl pivalate, chloromethyl pivalate or bromomethyl pivalate (employing a molar ratio of pivalate:II of within the range of from about 1:1 to about 10:1, preferably from about 2:1 to about 5:1) under an inert atmosphere such as argon. The reaction mixture is heated to a temperature within the range of from about 40 to about 50°C and amine base such as triethylamine, pyridine or diisopropylethylamine, and calcium salt such as CaCl₂, or calcium carbonate or other calcium salts, preferably CaCl₂, or calcium hydroxide, are added to form the pivaloyloxymethyl ester calcium salt III. The product is then isolated and purified by crystallization of the calcium salt from alcoholic solvents such as methanol, ethanol or isopropanol.

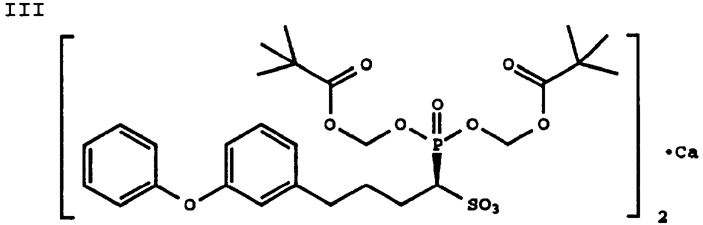

The amine base is employed in a molar ratio to II of within the range of from about 1:1 to about 5:1, and the calcium salt is employed in a molar ratio to free acid of within the range of from about 10:1 to about 100:1.

The calcium salt is passed through a cation-exchange resin to form the free acid.

An alcoholic (such as methanolic) solution of the free acid is treated with amine such as t-butylamine, t-octylamine or dehydroabietylamine employing a molar ratio of amine:III of within the range of from about 0.5:1 to about 2:1, preferably 1:1, to form the salt of the invention I.

The compounds of Formula I of the invention inhibit cholesterol biosynthesis by inhibition of de novo squalene production. These compounds inhibit the squalene synthetase enzyme and, in addition, some of the compounds of Formula I of the invention inhibit other enzymes in the pathway from isopentenyl diphosphate to squalene, that is, farnesyl diphosphate synthetase and isopentenyl diphosphatedimethylallyl diphosphate isomerase.

The compounds of the invention are useful in treating hyperlipoproteinemia, hyperlipidemia, hypercholesterolemia, hypertriglyceridemia, combined hypercholesterolemia and hypertriglyceridemia, and/or in preventing or inhibiting development of and/or treating atherosclerosis. Thus, the compounds of the invention may be used to treat diseases such as chylomicronemia syndrome, Type I hyperlipoproteinemia, familial combined hyperlipoproteinemia, familial hypertriglyceridemia, mixed hyperlipoproteinemia, familial hypercholesterolemia and Type III hyperlipoproteinemia and/or atherosclerosis.

In addition, the compounds of the invention may increase plasma high density lipoprotein cholesterol levels.

The compounds of the invention may also be useful in inhibiting formation of gallstones, treating hepatitis D (by virtue of protein prenyltransferase inhibition, Glenn et al, Science, Vol. 256, pp. 1331-1333, May 29, 1992), treating tumors, lowering blood pressure, lowering blood sugar, treating diabetes mellitus, treating inflammation, as a diuretic, as an inotropic agent, as an antiarthritic (antirheumatic) agent, in treating other diseases of calcium and phosphate metabolism including treatment of bone resorption, Paget's disease, osteoporosis, calcification of joints, implants and metastasis, as antitartar and anticalculus agents in toothpastes and mouthwashes, treating various stones and calculi, treating sickle cell anemia, treating hypoxia and ischemic tissue, and as an anti-ameobal agent, as well as for use in complexes with technetium-99m and radioiodinated derivatives for use as diagnostics.

U.S. application Serial No. 774,957 (EP-A-537007) discloses that post-translational modification of CAAX box containing proteins may be inhibited by administering a protein-prenyl transferase inhibitor which inhibits the transfer of the prenyl group [such as farnesyl (in the case of ras oncogene products), geranyl or geranylgeranyl] to the cysteine of the CAAX box by the protein-prenyl transferase enzyme. The protein-prenyl transferase inhibitor will block the protein-prenyl transferase enzyme from catalyzing the transfer of the prenyl group (for example, farnesyl, geranyl or geranylgeranyl) from the prenyl pyrophosphate to the cys residue of the CAAX box, such as the ras p21 cys, or to the CAAX box cysteine of other cAAX box containing proteins. In the case of ras p21 oncogene products, inasmuch as the cys is not farnesylated, in the presence of the protein prenyl transferase inhibitor, it cannot effect interaction of the ras protein with the membrane so that neoplastic transformation of the cell will be prevented. In this manner protein-prenyl transferase inhibitors prevent neoplastic transformation of the cell, thereby acting as an anti-cancer agent for the treatment of and/or prevention of ras-related tumors.

Examples of CAAX box containing proteins which have been demonstrated or are believed to undergo prenylation include, but are not limited to, ras, nuclear lamins, α or γ subunits of heterotrimeric G-proteins, γ-subunits of retinal transducin, G25K and K-rev p21, and protein families including rho, rap, rac, ral, and rab.

The compounds of the invention may be employed in a method for blocking or preventing the prenylation of CAAX box containing proteins such as ras oncogene products, and thereby inhibit disease promoting effects of the CAAX box containing protein or more specifically prevent and/or treat ras-related tumors, by administering to a patient in need of treatment a therapeutic amount of a compound of Formula I of the invention which serves as a protein-prenyl transferase inhibitor.

The Formula I protein-prenyl transferase inhibitors, unlike HMG CoA reductase inhibitors, will interfere with prenylation of the ras oncogene products and inhibit their transforming activity, yet may or may not interfere with the synthesis of FPP, a precursor in the synthesis of ubiquinones, dolichols and Haem A.

The compounds of the invention may also be employed in combination with an antihyperlipoproteinemic agent, hypocholesterolemic agent, and/or hypotriglyceridemic agent, and/or antiatherosclerotic agent such as one or more HMG CoA reductase inhibitors, for example, pravastatin, lovastatin, simvastatin, velostatin, fluvastatin, rivastatin, compactin, SDZ-63,370 (Sandoz), CI-981 (W-L), HR-780, L-645,164, CL-274,471, dalvastatin, α-, β-, and γ-tocotrienol, (3R,5S,6E)-9,9-bis(4-fluorophenyl)-3,5-dihydroxy-8-(1-methyl-1H-tetrazol-5-yl)-6,8-nonadienoic acid, L-arginine salt, (S)-4-[[2-[4-(4-fluorophenyl)-5-methyl-2-(1-methylethyl)-6-phenyl-3-pyridinyl]ethenyl]hydroxy-phosphinyl]-3-hydroxybutanoic acid, disodium salt, BB-476, (British Biotechnology), dihydrocompactin, [4R-[4α,6β(E)]]-6-[2-[5-(4-fluorophenyl)-3-(1-methylethyl)-1(2-pyridinyl)-1H-pyrazol-4-yl]ethenyl]tetrahydro-4-hydroxy-2H-pyran-2-one, and/or 1H-pyrrole-1-heptanoic acid, 2-(4-fluorophenyl)β,δ-dihydroxy-5-(1-methylethyl)-3-phenyl-4-[(phenylamino)carbonyl]calcium salt[R-(R*,R*)]; one or more fibric acid derivatives such as clofibrate, bezafibrate, Lopid(gemfibrozil) one or more other cholesterol biosynthesis inhibitors, such as NB-598, N-(1-oxododecyl)-4α,10-dimethyl-8-aza-trans-decal-3β-ol, 2,4-undecadienoic acid, 11-[3-(hydroxymethyl)-4-oxo-2-oxetanyl]-3,5,7-trimethyl-, [2R-[2α(2E,4E,7R*),3β]]; one or more bile acid sequestrants, for example, cholestyramine, colestipol, polidexide (DEAE-Sephadex); one or more antioxidants, for example probucol and Vitamin E; and/or one or more other lipid lowering and/or antiatherosclerotic agents, for example nicotinic acid or derivatives thereof, neomycin, p-aminosalicylic acid, probucol, hydroxy-propylmethylcellulose, LS-2904, ethanol, 2-[[1-methyl-2-[3-(trifluoromethyl)phenyl]ethyl]amino]benzoate (ester).

The above compounds to be employed in combination with the squalene synthetase inhibitor of the invention will be used in amounts as indicated in the Physicians' Desk Reference (PDR).

The compounds of the invention may also be employed with sodium lauryl sulfate of other pharmaceutically acceptable detergents to enhance oral bioavailability of such compounds.

Inhibition of squalene synthetase may be measured by the following procedure.

Rat liver microsomal squalene synthetase activity is measured using farnesyl diphosphate as substrate and quantitating squalene synthesis using gas chromatographic analysis. The assay was developed by modifying conditions originally described by Agnew (Methods in Enzymology 110:357, 1985).

A further aspect of the present invention is a pharmaceutical composition consisting of at least one of the compounds of the invention, such as Formula I, in association with a pharmaceutical vehicle or diluent. The pharmaceutical composition can be formulated employing conventional solid or liquid vehicles or diluents and pharmaceutical additives of a type appropriate to the mode of desired administration. The compounds can be administered to mammalian species including humans, monkeys, dogs, etc., by an oral route, for example, in the form of tablets, capsules, granules or powders, or they can be administered by a parenteral route in the form of injectable preparations. The dose for adults is preferably between 200 and 2,000 mg per day, which can be administered in a single dose or in the form of individual doses from 1-4 times per day.

A typical capsule for oral administration contains active ingredient (250 mg), lactose (75 mg) and magnesium stearate (15 mg). The mixture is passed through a 60 mesh sieve and packed into a No. 1 gelatin capsule.

A typical injectible preparation is produced by asceptically placing 250 mg of sterile active ingredient into a vial, asceptically freeze-drying and sealing. For use, the contents of the vial are mixed with 2 mL of physiological saline, to produce an injectible preparation.

The following Examples represent preferred embodiments of the present invention.

### Example 1

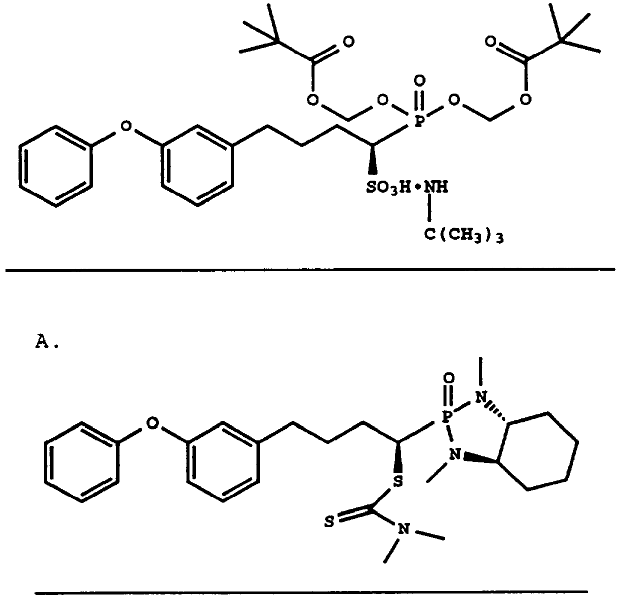

The title Part A compound was prepared as described in Example 178 of European Patent Application No. 0595635A1.

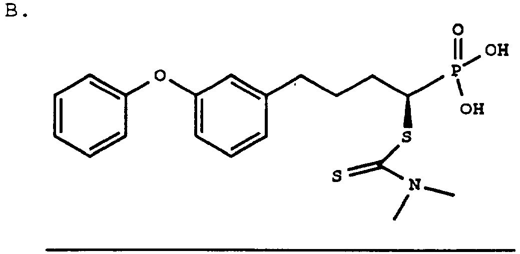

3 N Hydrochloric acid solution (124 mL) was added dropwise to a stirred solution of Part A compound (6.14 grams, 11.8 mmol) in acetonitrile (124 mL) at room temperature in about 10 minutes. The resulting mixture was stirred for 3 hours to complete the reaction. The reaction mixture was concentrated on a rotary evaporator to remove most of the acetonitrile. The remaining aqueous layer was extracted with ethyl acetate (3 x 100 mL). The combined organic layer was washed with water (2 x 50 mL), brine (50 mL) and dried over magnesium sulfate and filtered. The filtrate was concentrated to dryness on a rotary evaporator to afford the crude title Part B compound as an oil (4.83 grams, 87% yield). This compound was used in the next reaction without any further purification.

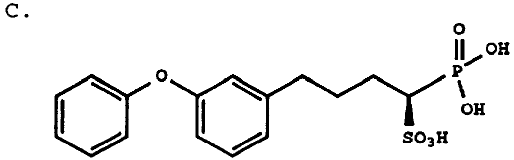

To the stirred crude Part B compound (4.83 grams, 11.35 mmol) in glacial acetic acid (40 mL) was added formic acid (4.5 mL) and cooled to ∼10°c. To this mixture, 30% aqueous hydrogen peroxide (6.97 mL, 68.1 mmol) was very slowly added in ∼15 minutes keeping the internal temperature of the reaction below 30°C. A yellow polymeric solid precipitated out from the reaction. The reaction mixture was slowly warmed to room temperature in about 20 minutes and then stirred for six hours to complete the reaction. Excess peroxide was carefully decomposed by the slow addition of dimethyl sulfide (2.08 mL, 2.5 equiv.). After stirring for 30 minutes, the mixture was filtered through a sintered funnel to remove the polymeric solid. The filtrate was concentrated on a rotary evaporator to afford a residue. The residue was dissolved in a mixture of ethyl acetate-toluene (1:5) and concentrated on a rotary evaporator. This procedure was repeated four times to remove most of the acetic acid to yield the crude title Part C compound.

The crude title compound was purified by two methods:

### Method (1)

The crude title C was dissolved in methanol (15 mL) and treated with an ethyl acetate solution (150 mL) of 1-adamantanamine (3.54 grams, 22.69 mmol) at room temperature. After stirring for an hour the precipitated bis-adamantanamine salt of the title compound was collected on a Buchner funnel. The white solid was washed with ethyl acetate (50 mL) and dried in a vacuum oven at room temperature to afford 7.60 grams. To a suspension of this salt in methanol (-75 mL), AG® 50W-X8 Ion-Exchange resin (55 grams) was added and stirred at room temperature for 30 minutes. The mixture was then poured into a sintered funnel containing AG® 50W-X8 Ion-Exchange resin (25 grams). The methanolic filtrate was concentrated on a rotary evaporator to afford purified title compound (3.8 grams, 83% overall yield from Part A compound)

### Method (2)

The crude title triacid C (4.93 grams) was dissolved in a mixture of ethyl acetate (30 mL) and methanol (∼5 mL) at reflux. To this stirred solution was added cinchonidine (4.43 grams, 2.0 equiv.) in ethyl acetate (30 mL). The mixture was heated to reflux to obtain a homogeneous solution. The solution was allowed to cool to room temperature where crystals of title triacid bis-cinchonidine salt were formed. The crystals after cooling at 4°C for ∼14 hours were collected on a Buchner funnel. First crop afforded 2.33 grams and a second crop (2.49 grams) was obtained from the mother liquor. The combined cinchonidine salt (4.82 grams) was dissolved in methanol (50 mL) and treated with Dowex®50X8 Ion-Exchange resin (50 grams). The suspension was stirred for an hour and filtered. The filtrate was concentrated to afford purified title compound.

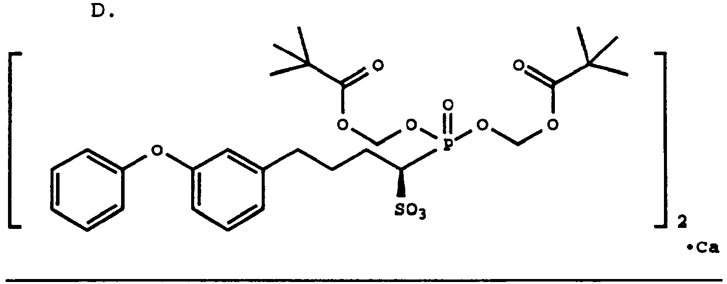

To a stirred solution of Part C compound (1.045 g, 2.7 mmol) in anhydrous DMF (9 mL) at room temperature was added iodomethyl pivalate (2.59 g, 10.70 mmol) under argon and the mixture was heated (internal temperature ranged between 41.5-45.9°C). Triethylamine (0.9 mL, 6.46 mmol) was slowly added in small portions in about an hour. After stirring for about 15 minutes an aliquot from the reaction mixture was analyzed by HPLC. The reaction was not complete. Iodomethyl pivalate (0.962 g, 3.97 mmol) in one portion followed by triethylamine (0.23 mL, 1.65 mmol), in about 30 minutes, were added and the reaction continued for another 30 minutes and then the reaction monitored by HPLC. Additional triethylamine (0.035 mL, 0.25 mmol) was added to complete the reaction. The reaction mixture was poured into a separatory funnel containing 5% sulfuric acid (10 mL). Water (∼10 mL) was used to rinse the remaining contents of the reaction flask into the separatory funnel. The mixture was washed with hexanes (5 x 40 mL) [hexanes layers containing the iodomethyl pivalate was discarded] and extracted with ethyl acetate (2 x 40 mL). The combined ethyl acetate layer was washed with 5% Na₂S₂O₃ (2 x 40 mL), saturated NaHCO₃ (3 x 40 mL) and finally with 2 M cacl₂ (4 x 40 mL). The organic layer was concentrated on a rotary evaporator to afford the crude product as an off-white foamy solid. It was dissolved in absolute ethanol (16 mL) and left for crystallization at room temperature for 12 hours. The crystallized product was filtered (after keeping at 4°C for ∼1 hour) on a Buchner funnel and the solid was washed with ethanol (2 x 2 mL) and dried under high vacuum to afford title compound (1.18 g in -70% yield).

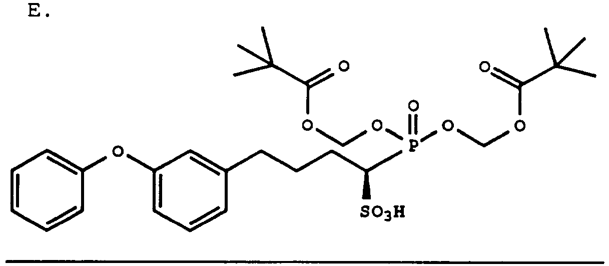

AG® 50W-X8 Ion-Exchange resin (95 grams) was added in one portion to a stirring solution of Part D calcium salt (9.53 grams, 15.04 mmol) in methanol (480 mL). The heterogeneous mixture was stirred for 30 minutes and filtered through a pad of AG® 50W-X8 Ion-Exchange resin (25 grams). The resin was washed with methanol (∼100 mL). The combined methanolic solution containing title free acid was used as such to make different salts.

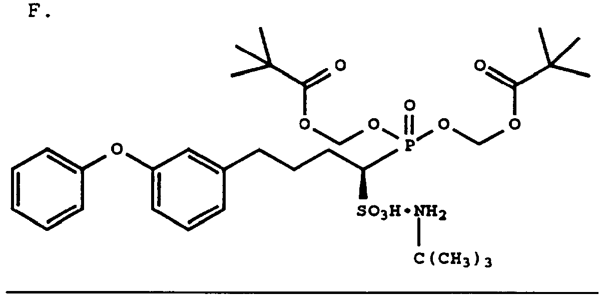

To a stirring methanolic (580 mL) solution of Part E free acid (prepared from 9.53 grams (15.04 mmol) of Example 1 Part D calcium salt) was added t-butylamine (1.10 grams, 15.04 mmol) very slowly. After stirring for 15 minutes the reaction mixture was concentrated on a rotary evaporator to afford a foamy solid. The residue was dissolved in ethyl acetate (9 mL) and hexanes (45 mL) and the mixture was left at room temperature to crystallize the salt. After 12 hours the crystals were collected on a Buchner funnel and washed with a mixture of ethyl acetate and hexanes (1:9, ∼40 mL) and dried under vacuum to afford title t-butylamine salt as a white solid (9.3 grams).

### Example 2

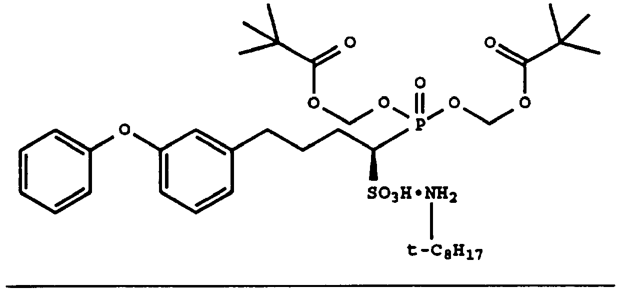

To a stirring methanolic (7 mL) solution of Example 1 Part E free acid (prepared from 0.15 grams (0.241 mmol) of Example 1 Part D calcium salt) was added t-octylamine (0.033 grams, 0.241 mmol) very slowly. After stirring for 15 minutes the reaction mixture was concentrated on a rotary evaporator to afford a foamy solid. The residue was dissolved in ethyl acetate (0.5 mL) and hexanes (2.5 mL) and the mixture was left at room temperature to crystallize the salt. After 12 hours the crystals were collected on a Buchner funnel and washed with a mixture of ethyl acetate and hexanes (1:9, ∼5 mL) and dried under vacuum to afford title t-octylamine salt as a white solid.

### Example 3

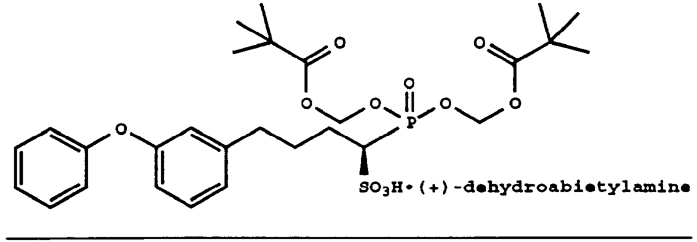

To a vigorously stirring methanolic (20 mL) solution of Example 1 Part D free acid (prepared from 0.64 grams (∼1 mmol) of Example 1 Part C calcium salt) was added deionized water (150 mL) followed by slow addition of (+)-dehydroabietylamine (0.29 grams, 1.0 mmol) in methanol (5 mL). A precipitate was formed during the addition. After stirring for an hour the precipitate was collected on a Buchner funnel. The solid was washed with deionized water (100 mL) and dried under vacuum to afford title salt as a white solid (0.6 grams).

## Claims

1. A phosphonosulfonate salt having the structure 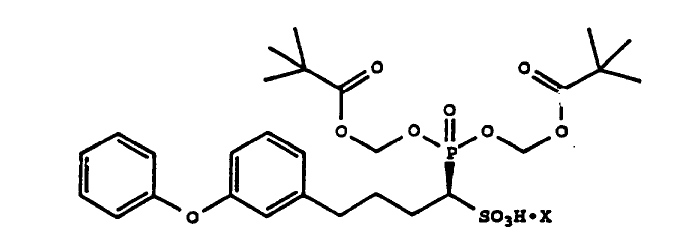 wherein X represents the t-butylamine salt, the t-octylamine salt or the dehydroabietylamine salt.

2. The salt as defined in Claim 1 which is the t-butylamine salt.

3. The salt as defined in Claim 1 which is the t-octylamine salt.

4. The salt as defined in Claim 1 which is the dehydroabietylamine salt.

5. A pharmaceutical composition comprising a phosphonosulfonate salt as defined in any preceding claim and a pharmaceutically acceptable carrier therefor.

6. Use of a compound having the structure 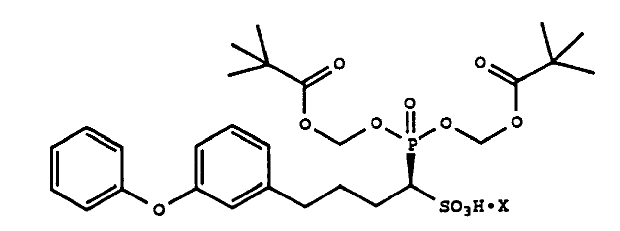 wherein X represents the t-butylamine salt, the t-octylamine salt or the dehydroabietylamine salt, for the manufacture of a medicament for inhibiting cholesterol biosynthesis, lowering serum cholesterol, or inhibiting and/or treating hyperlipemia, hyperlipidemia, hyperlipoproteinemia, hypercholesterolemia, and/or hypertriglyceridemia, or inhibiting and/or treating atherosclerosis.

7. The use as defined in Claim 6 wherein the salt is the t-butylamine salt.

8. The use as defined in Claim 6 wherein the salt is the t-octylamine salt.

9. The use as defined in Claim 6 wherein the salt is the dehydroabietylamine salt.

10. The use as defined in Claim 6 wherein the medicament is for treating atherosclerosis.

11. The use as defined in Claim 6 wherein the medicament is for treating hypercholesterolemia.

12. A method for isolating and purifying the free acid of the following structure in substantially purified form 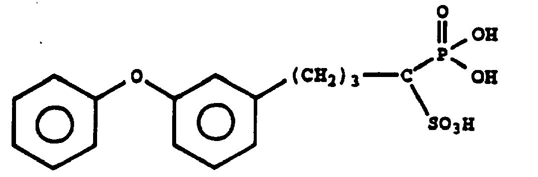 which comprises reacting the free acid with 1-adamantanamine or cinchonidine, to form the corresponding salt, and subjecting the so-formed salt to ion exchange to afford substantially purified free acid.

13. The method as defined in Claim 12 wherein 1-adamantanamine is reacted with the free acid to form the corresponding bis-adamantanamine salt of said free acid.

14. The method as defined in Claim 12 wherein cinchonidine is reacted with the free acid to form the corresponding bis-cinchonidine salt.

15. The method as defined in Claim 12, 13 or 14 where the free acid is initially dissolved in an alcohol solvent.

16. A method for forming a pivaloyloxymethyl calcium salt of a phosphonosulfonate of the structure 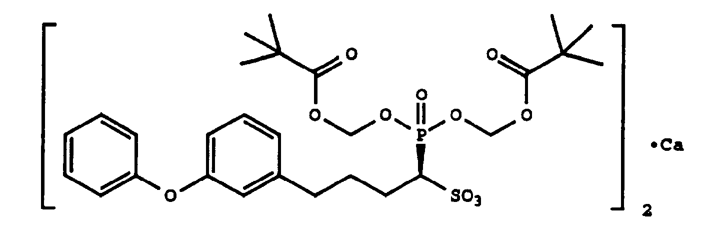 which comprises providing a solution phosphonosulfonate free acid of the structure 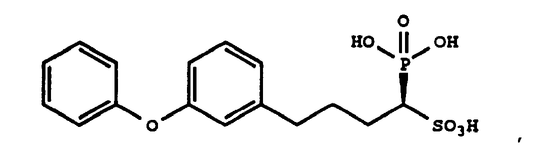 and treating the free acid with halomethyl pivalate under an inert atmosphere, in the presence of amine base, and with a calcium salt or hydroxide, to form the pivaloyloxymethyl calcium salt of the phosphonosulfonate.

17. The method as defined in Claim 16 wherein the halomethyl pivalate is chloro-, bromo- or iodomethyl pivalate.

18. A method for preparing an amine salt of a phosphonosulfonate of the structure 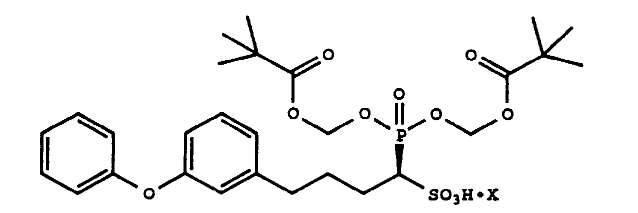 wherein X is an amine salt which is the t-butylamine salt, t-octylamine salt or dehydroabietylamine salt, which comprises providing a calcium salt of a phosphonosulfonate of the structure 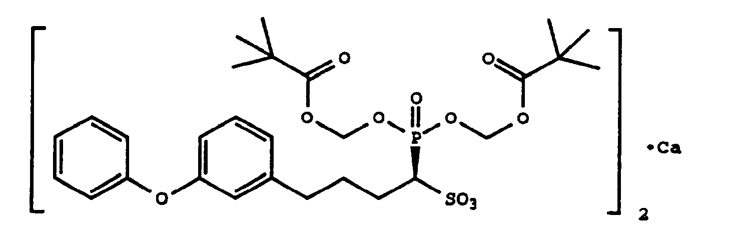 treating the calcium salt was an ion exchange resin to afford the free acid as defined above and reacting the free acid with an amine reactant to form the amine salt of the phosphonosulfonate as defined above.

19. The method as defined in Claim 18 wherein the amine reactant is t-butylamine, t-octylamine or dehydroabietylamine.

20. A compound of any one of claims 1-4 for use as an active pharmaceutical substance.
